# EUROPEAN PATENT SPECIFICATION

(11) **EP 0 767 700 B1**
(45) Date of publication and mention of the grant of the patent: **27.11.2002**
(21) Application number: 96915420.2
(22) Date of filing: 25.04.1996
(51) Int. Cl.: B01D 27/06, B01D 27/00

(54) **METHOD OF MANUFACTURING A FLUID FILTER**
METHODE ZUR HERSTELLUNG EINES FILTERS FÜR FLUIDEN
PROCEDE DE FABRICATION D'UN FILTRE POUR FLUIDES

(30) Priority: 27.04.1995 US 429829; 29.03.1996 US 626254
(43) Date of publication of application: 16.04.1997
(73) Proprietor: Avecor Cardiovascular Inc., Plymouth, MN 55441 (US)
(72) Inventor: PETERSON, Richard, O., Edina, MN 55424 (US); OLSEN, Robert, W., Plymouth, MN 55442 (US)
(74) Representative: Smulders, Theodorus A.H.J., Ir.
(86) International application number: US9606056
(87) International publication number: WO96033791

(56) References cited:
- EP-A- 0 200 798
- EP-A- 0 256 772
- US-A- 2 463 825
- US-A- 3 877 903
- US-A- 4 430 223
- US-A- 4 744 902
- US-A- 4 824 564
- US-A- 4 919 802

## Description

### FIELD OF THE INVENTION

This invention relates to blood filters used in extracorporeal blood circuits. More particularly, the invention is directed to an arterial line blood filter used during heart bypass surgery to filter solid particulate and gaseous emboli from blood that has been oxygenated and is being returned to the patient. This invention relates to a method of manufacturing a fluid filter, especially an arterial line blood filter.

### BACKGROUND OF THE INVENTION

During open heart surgery the blood of the patient is bypassed to an extracorporeal blood circuit. The circuit commonly includes a support system which supplies the pumping function of the heart and the oxygenation function of the lungs. This support system effectively isolates the heart and lungs, enabling the surgeon to make the necessary repairs to the heart and/or lungs. Both venous blood and cardiotomy blood from the surgical site may be removed and circulated through the extracorporeal circuit.

Blood filters are typically included both upstream and downstream from the oxygenator, which may incorporate a heat exchanger. Upstream blood filters include venous and cardiotomy filters through which the blood may be filtered prior to entering the oxygenator and which are used to remove particulate, especially from the surgical site, and bubbles from the blood. Although these upstream filters are effective, it is possible that some emboli may pass through or be generated in the oxygenator and/or heat exchanger. These emboli may be in suspension in the oxygenated blood which is to be returned to the patient. The embolic material can be either particulate, such as platelet or white cell aggregates, or gaseous, such as small or large gas bubbles. Therefore, an arterial line filter which is located downstream of the oxygenator is critically important in trapping and removing any remaining emboli before the blood is provided to the patient.

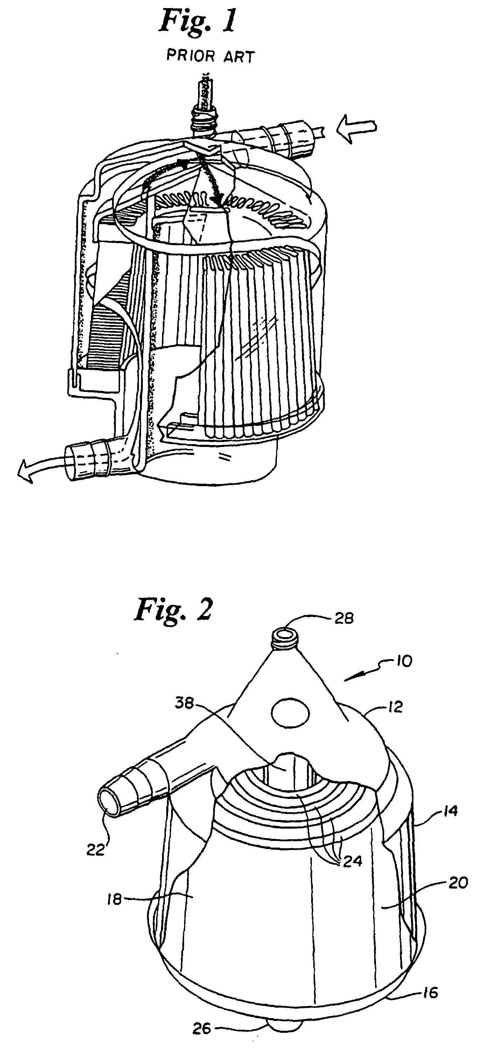

A typical conventional arterial blood filter is illustrated in FIG. 1. The filter includes a blood inlet located at the top of the filter. In this case the inlet is located in a tangential position. A radially pleated tubular filter is disposed within a cylindrical housing and is covered by a conically shaped cap. The arrow shows the path of blood as it flows through the filter. After entering through the tangential inlet the blood travels in a circular path over and around the cap and down between the outer wall of the housing and the pleated filter. The blood eventually passes through the filter and is discharged through a blood outlet at the bottom of the device. A gas port is located at the top of the device through which air bubbles or gaseous emboli may be vented.

Although conventional arterial blood filters are effective in achieving significant reductions in emboli, they share several common problems. First, they include significant horizontal surface area in the blood flow path. Bubbles tend to attach themselves to these horizontal surfaces, increasing the difficulty of priming and, therefore, making the filters more difficult to use. Second, in many radially pleated designs, blood flows down through a small annular gap between the filter element and the housing. At a given flow rate, the smaller this area is, the higher is the downward blood velocity. Higher blood velocities entrain smaller air bubbles which subsequently get trapped in the filter element or get broken up into even smaller bubbles and pass through the filter. It would be desirable to have as large an area as possible available for downward flow to reduce downward entrainment of small air bubbles. Third, in many conventional arterial filters the priming volume is not utilized efficiently. Generally, the bigger the prime volume of a filter, the longer the time available for air bubbles to separate because of their buoyancy. However, there is also a desire to minimize prime volume to avoid unnecessary use of blood and blood products. The best balance is achieved by ensuring that as much of the prime volume as possible is upstream of the filter screen, because this is the region in which air can separate by buoyancy. Fourth, in most radially pleated designs the frontal area for flow downstream of the filter element is substantially reduced from the frontal area upstream of the filter element. Thus, the blood velocity significantly increases after it passes through the filter element in conventional designs. This higher velocity tends to entrain bubbles which have passed through the filter element and carries them toward the outlet port, where the bubbles may be conducted to the patient. It is desirable to maintain the frontal area for flow adjacent to the downstream side of the filter element as large as possible, or at least as close as feasible to the frontal area upstream of the filter element. Fifth, radially pleated designs incorporate a shallow annular or disk-shaped potting cup to mount the top and bottom of the filter element. The filter element end is immersed in the liquid potting compound and held in position until the compound solidifies. Since the cup is rarely completely filled with the potting compound, a concave surface is formed by the potting compound and the lip of the cup. Concave surfaces can also be created by the liquid potting compound wicking up the filter element while the compound is hardening from a liquid to a solid. In use, bubbles can become trapped under the concave surface of the upper potting cup. When the filter is inverted during priming to remove these trapped bubbles, they can rise and be trapped under the concave surface of the bottom potting cup which is now on top. This bubble trapping complicates priming the filter. It is desirable to create a design which does not require potting either the top or bottom of the filter element. Thus, it would be desirable to provide an arterial blood filter which not only has efficient emboli removal capabilities, but is also easier to prime and provides more opportunity for buoyancy separation of air than do conventional filters.

### SUMMARY OF THE INVENTION

In accordance with the present invention there is disclosed a method of manufacturing a fluid filter element. The method comprises providing a cone having a tip end and an open end and made of a filter material; providing a support element having a plurality of concentric annular members mounted on a base and having outermost and innermost concentric annular members; aligning the central axes of the cone and the support element with the tip end of the cone closest to the support element; inserting each of a plurality portions of the cone into separate circular areas between two concentric annular members by placing a plurality of forming sleeves inside the cone and inserting the forming sleeve between the two concentric annular members, the concentric annular members being generally cylindrical and having an interior and an exterior; inserting a portion of the cone into the circular area in the interior of the innermost annular member by a forming sleeve; and forming a portion of the cone over the exterior of the outermost annular member by inserting a forming sleeve outside the outermost annular member.

In an alternative embodiment, the method comprises providing a cone having a tip end and an open end and made of a filter material; providing a support element having a plurality of concentric annular members mounted on a base and having outermost and innermost concentric annular members; aligning the central axes of the cone and the support element with the open end of the cone closest to the support element; inserting each of a plurality of portions of the cone into separate circular areas between two concentric annular members by placing a plurality of forming sleeves over the cone and inserting the forming sleeve between the two concentric annular members, the concentric annular members being generally cylindrical and having an interior and an exterior; inserting a portion of the cone into the circular area in the interior of the innermost annular member by a forming sleeve; and forming a portion of the cone over the exterior of the outermost annular member by inserting a forming sleeve outside the outermost annular member.

### BRIEF DESCRIPTION OF THE DRAWINGS

The foregoing and other aspects of the present invention will be best appreciated with reference to the detailed description of the invention, which follows, when read in conjunction with the accompanying drawings wherein:
FIG. 1 is a partially cut away perspective view of a typical prior art arterial blood filter.
FIG. 2 is a perspective view of an arterial blood filter manufactured according to the present invention with a portion of the housing broken away to show the concentric pleats of the filter element.
FIG. 3 is a cross-sectional side view of the arterial blood filter of FIG. 2.
FIG. 4 is a top view of the cap of the arterial blood filter of FIG. 2.
FIG. 5 is a bottom view of the cap of FIG. 4.
FIG. 6 is a perspective view of the support element utilized in the arterial blood filter of FIG. 2.
FIG. 7 is a top view of the support element of FIG. 6.
FIG. 8 is a sectional view of the support element taken along line 8-8 of FIG. 7.
FIG. 9 is a front view of a flat pattern for a filter cone.
FIGS. 10 to 14 are cross-sectional side views of steps in the manual pleating of the filter cone, with the pleats formed inside pleat first.
FIGS. 15 to 17 are cross-sectional side views of steps in the manual pleating of the filter cone, with the pleats formed outside pleat first.
FIGS. 18 to 23 are cross-sectional side views of steps in the automated pleating of the filter cone.
FIG. 24 is a cross-sectional side view of the step of ultrasonically staking the filter cone to the support element.
FIG. 24a is a magnified detail of FIG. 24.
FIG. 25 is a cross-sectional side view of the step of cutting the excess cone material away from the support element.
FIG. 26 is a cross-sectional side view of the lower portion of the filter.

### DETAILED DESCRIPTION OF THE INVENTION

### Construction of the Blood Filter

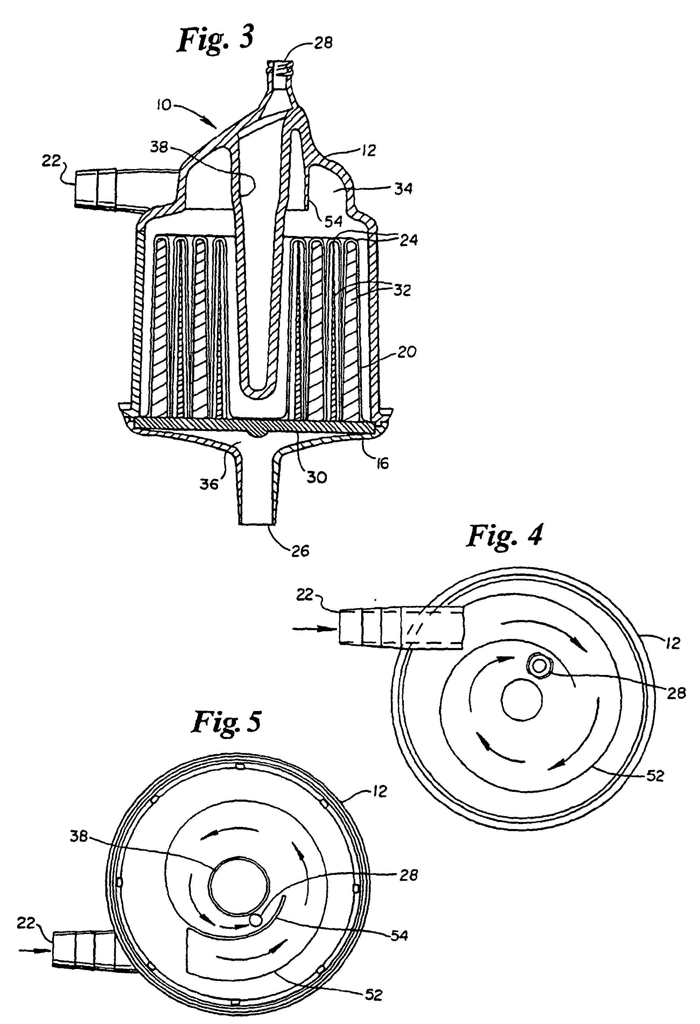

FIGS. 2 and 3 are perspective and sectional views of an arterial blood filter 10 manufactured in accordance with the present invention. Blood filter 10 has a housing which includes a cap portion 12, a tubular wall portion 14 and a base portion 16. The housing encloses a blood filtering chamber 18 which contains a filter element 20 having a plurality of concentric pleats 24. As will be discussed in more detail with respect to FIGS. 3 and 6 to 8, filter element 20 is supported by a support element, not shown in FIG. 2. Cap portion 12, wall portion 14 and base portion. 16 may be formed from separate pieces and bonded together in a conventional manner. Alternatively, wall portion 14 may be constructed as an integral piece with either cap 12 or base portion 16. In the preferred embodiment shown in FIGS. 2 and 3 the cap portion 12 is integral with wall portion 14. Preferably, the housing is made of a transparent medical grade material so that the user is able to observe the flow of blood through the device.

A blood inlet 22 is located at an upper position on the housing to enable blood to be circulated through the blood filtering chamber 18. Blood inlet 22 may be connected to a line from an oxygenator (not shown) for receiving oxygenated blood. In the embodiment shown in FIG. 2 the blood inlet is formed as an integral portion of cap 12. Inlet 22 is constructed such that when the filter is placed in its upright position with the cap at the top, as shown in FIG. 2, the blood enters horizontally at one side of the blood filter. After the blood enters the blood filter it is directed by the cap in an inwardly spiral path, as will be discussed in more detail with respect to FIGS. 4 and 5. A blood outlet 26 is located at the bottom of the housing in the base portion 16. The outlet 26 is connected to a patient return line (not shown) for providing oxygenated blood back to the patient. A gas vent 28 is located at or near the top of cap portion 12. Vent 28 provides a means of venting from the blood filter gaseous emboli which rise to the top of cap portion 12. The vent 28 is located at the highest point of cap 12.

FIG. 3 is a cross-sectional side view of the arterial blood filter of FIG. 2. A support element 30 is positioned within the housing to provide support for the concentric pleats 24 of filter element 20. Generally, any manner of support which results in stabilizing the annular pleats may be used. Preferably, support element 30 includes a plurality of concentric annular members 32, each larger in diameter than the previous, which nest within the concentric pleats 24. Each annular member is a generally cylindrical tubular section. As shown in FIG. 3, the pleats are generally concentric to the longitudinal axis of the blood filter and spaced apart from the surrounding wall portion and each other to leave sufficient space for blood to flow down and through the filter member. Inside of the innermost concentric pleat is a volume displacer 38. Volume displacer 38 is a closed end tube which extends from the cap portion 12 towards the base portion 16. It may be formed as an integral part of cap portion 12, as shown, or it may be a separate piece which is bonded to the cap portion. Although volume displacer 38 is optional, its use is desirable since it serves the important function of lowering the priming volume of the blood filter.

### The Concentric Pleated Filter Element

The concentric pleats of the filter element are formed from a conventional filter screen fabric. The filter screen fabric is cut and one or more seams bonded so the resulting shape is a hollow cone. The axis of the pattern may be cut on the bias of the screen threads. A bias cut allows the screen to conform better to the desired shape without forming wrinkles, creases, or bulges. The cone may be formed into concentric pleats by shaping the screen over the annular members of the support element using cylindrical forming sleeves.

The filter element 20 is bonded along the outer periphery of the support element 30. The filter screen is also bonded to each spoke of the filter support element to prevent the screen cone from inverting if flow through the filter is retrograde. Support element 30 is in turn bonded to the base portion 16 such that when the blood filter is assembled, filter element 20 divides blood filtering chamber 18 into a blood inlet chamber 34 and a blood outlet chamber 36.

With continued reference to FIG. 3, it will be appreciated that the length of the annular members 32 of the support element 30 is approximately the same as the length of the wall portion 14 between the cap portion 12 and the base portion 16. Thus, the pleated filter element formed over the support element approximately fills the blood filtering chamber between the cap portion and base portion of the housing. This configuration is desirable in order to maximize the amount of surface area of the filter element which is exposed to blood flow. Additionally, the support element 30 and its annular members 32 provide the added benefit of displacing additional volume, resulting in the further lowering of the priming volume of the blood filter on the downstream side of the filter screen.

The number of annular pleats (and consequently the number of annular members) is selected to maximize the surface area of the filter element which is exposed to blood flow while at the same time leaving enough space between adjacent pleats, the wall portion and the volume displacer so that efficient blood flow may be maintained. In the preferred embodiment shown, four pleats are used. However, it will be appreciated by those of skill in the art with knowledge of the present invention that fewer or more pleats may be used within the scope of the invention.

### The Support Element

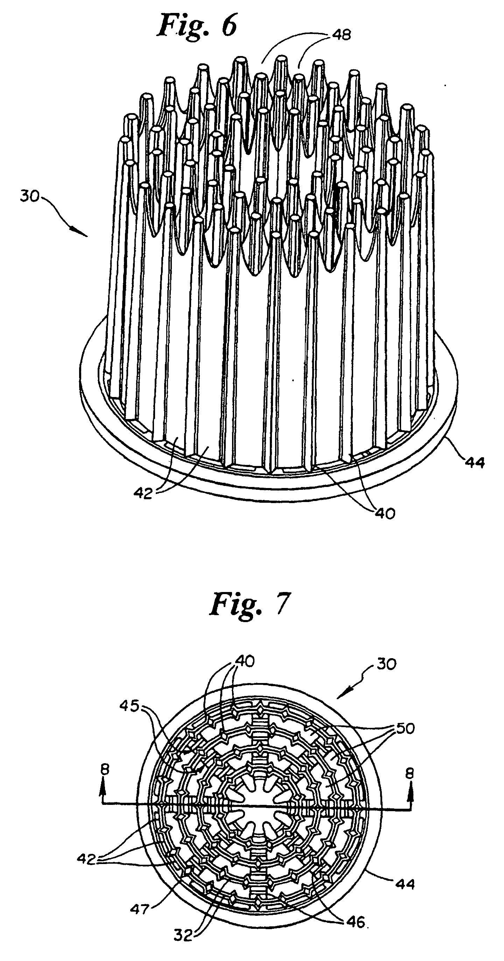

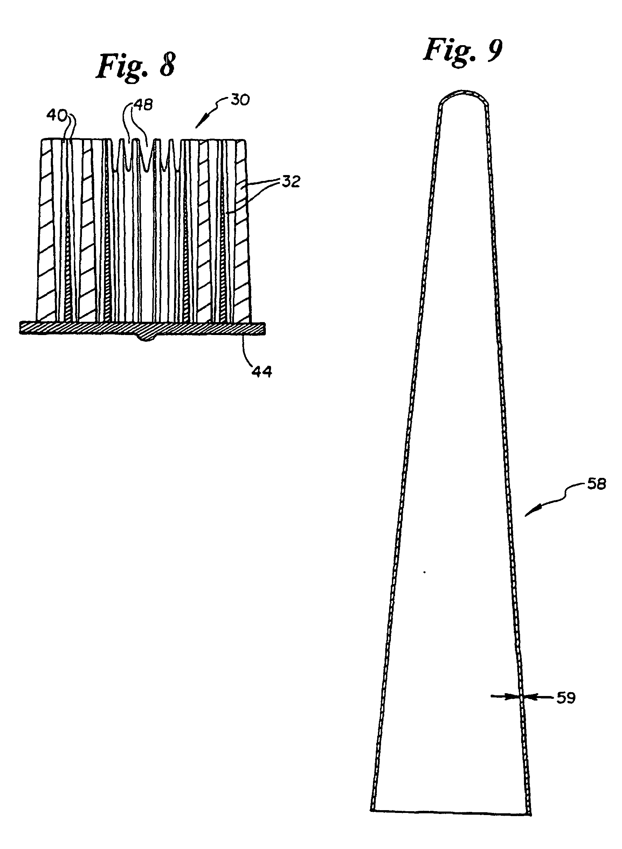

Support element 30 is shown in more detail in FIGS. 6 to 8. FIG. 6 is a perspective view of the support element showing the concentric ringed configuration of the annular members. FIG. 7 is a top view of the support element. FIG. 8 is a cross-sectional view of the support element taken along line 8-8 of FIG. 7. Support element 30 includes a base ring 44. Base ring 44 provides support for a network of spokes 46 from which the annular members 32 project. Each of the components including base ring 44, spokes 46 and annular members 32 may be made separately and then bonded together in a conventional manner to form support element 30. Preferably, support element 30 is molded from a medical grade plastic as a single integral piece which is bonded in a conventional manner to base portion 16. Alternatively, support element 30 may be formed as an integral portion of base portion 16.

In order to hold the filter element off of the surface of the annular members, ribs 40 may be provided on the inner and/or outer cylindrical surfaces of one or more of the annular members. In the preferred embodiment shown in the figures, the ribs are diamond shaped in cross-section, are generally parallel to the longitudinal axis of the blood filter and protrude from both the inner and outer cylindrical surfaces of each annular member. The number of ribs and their orientation with respect to the surface of the annular members may be varied. For example, the ribs may project from either the inner cylindrical surface or the outer cylindrical surface or both, as shown. Likewise, the ribs need not be parallel to the axis of the blood filter. Preferably, the ribs on the outer surface of one cylindrical annular member do not line up with the ribs on the inner surface of the adjacent annular member. This facilitates the molding process since it allows the metal in the mold to be thicker at the base of the support element where the ribs are in closest proximity. This enables the mold to be made stronger and more durable. It also conducts heat more rapidly so it cools more quickly after the plastic is injected during the molding process. The ribs provide a path between the filter element and the cylindrical surface of the annular members for blood which has passed through the filter element to flow from the outlet chamber to the blood outlet.

The cylinders which form the annular members may comprise flat planar facets 42 between ribs 40 such that the resultant shape is a polyhedron. Such a configuration maximizes the gap between the filter element and the inner and outer surfaces of the facets. This further ensures that the blood flow path between the filter element and the surfaces of the facets is adequate. In order to minimize the surface area of the filter element which is blocked off by contact with the upper edge of the annular members, the top edge of the facets 42 may be provided with notches 48. Notches 48 allow blood to flow through the filter element at the top of the pleats in areas that would be blocked in the absence of the notches. After passing through the filter element, the blood flows in a downward direction through outlet slots 50 formed between spokes 46 and annular members 32 and out of the blood filter through blood outlet 26.

### Inlet Blood Flow Path

The path followed by blood from the inlet 22 is best shown in FIGS. 4 and 5. FIGS. 4 and 5 are top and bottom views of cap portion 12, respectively. As illustrated by the arrows, the blood is directed to flow in an inward spiral by the inner surface 52 of cap portion 12. The tightness of the spiral increases along the flow path as the blood is directed around the volume displacer 38 inwardly towards the center of the blood filter. A flow director 54 is provided to separate blood which has circled around the blood filter from blood just entering through the blood inlet. Such interaction could reduce the cross sectional area available for flow of the inlet blood. This would increase the blood velocity in this region, which can increase the pressure losses in the inlet, increase damage to the blood and disrupt the even supply of blood over the pleats of the filter element. In addition, the flow director prevents bubbles which are rising to the vent port from mixing with blood entering from the inlet which is traveling at a high velocity. This prevents the high velocity inlet blood flow from breaking up bubbles rising to the vent port and from entraining those bubbles.

### Operation of the Blood Filter

In use, blood enters the inlet chamber through blood inlet 22. The blood flow is directed by the shape of inner surface 52 of the cap portion 12 in an inward spiral. Since the cross-sectional flow area in the inlet chamber is greater than the cross-sectional area of the inlet tubing, the inlet chamber acts as a diffuser and decreases the velocity of the blood. As the blood continues its flow at a decreased velocity around the cap portion of the blood filter, some blood begins to flow downward into the concentric annular pleats. Because the blood velocity is decreased and the blood is directed inwardly, there tends to be an even distribution of blood over the pleats.

As the blood follows its path through the filter, gaseous emboli rise to the top surface of the cap portion which is sloped upward and toward the vent. The inward flow path of blood carries the emboli around the spiral to the elevated region of the vent where they collect for venting. The elevated region of the vent is above the blood flow path so that the area is protected from high velocity blood flows which would flush the gaseous emboli out of the elevated vent region.

As the blood is distributed over the concentric pleats it passes through the filter element into the output chamber. The blood is then directed by the cylindrical annular members downward and through the output slots. From there it exits the blood filter through the blood outlet where it may be delivered to a patient through a patient return line.

### Method of Manufacturing a Fluid Filter

The invention provides a method of manufacturing a fluid filter as defined in the claims.

In a preferred embodiment, the forming sleeve is an annular forming sleeve. Other types of forming sleeves such as those having a plurality of prongs that may be inserted into the support element may also be used. In one embodiment, a second portion of the cone is inserted into the circular area in the interior of the innermost annular member by a forming sleeve; in another embodiment, a second portion of the cone is formed over the exterior of the outermost annular member by a forming sleeve inserted outside the outermost annular member. In a preferred embodiment, a second portion of the cone is inserted into the area in the interior of the innermost annular member and a third portion of the cone is formed over the exterior of the outermost annular member by inserting a forming sleeve outside the outermost annular member.

In an embodiment of the invention, the portions of the cone inserted into the support element are inserted substantially all of the distance to the base of the support element. In a preferred embodiment, each portion of the cone which is inserted into the support element is inserted all of the distance to the base of the support element.

In another embodiment, each of a plurality of portions of the cone is inserted into separate circular areas between two concentric annular members by a plurality of forming sleeves. In a preferred embodiment, the portions of the cone that are inserted into the support element are inserted in order from the innermost to outermost circular areas.

In another embodiment of the invention, one or more of the portions of the cone which are inserted into the support element are attached to the base of the support element. However, it is not necessary to attach the cone to the base of the support element; the filter performs well even if the cone is just pleated over the support element. The portions of the cone may be attached to the base of the support element by ultrasonic vibrations (ultrasonic staking), by inserting a portion of the cone into a groove on the base of the support element, or by an adhesive. In a preferred embodiment, the portions of the cone that are inserted into the support element are attached by inserting a portion of the cone into a groove on the base of the support element, and then subjecting the base of the support element to ultrasonic vibrations. In a preferred embodiment, the ultrasonic vibrations are conducted through energy directors. In another preferred embodiment, the support element has a lower melting temperature than the cone.

The cone also may be attached to the base of the support element by one or more additional support elements placed on top of the cone and support element. The one or more additional support elements may be placed outside the outermost member, or inside the concentric annular members. These one or more additional support elements preferably interlock with the support element.

In one embodiment, the base of the support element is cylindrical. In preferred embodiments of the invention, the support element has from two to six concentric annular members, more preferably from two to four concentric annular members. In another embodiment, the support element has four concentric annular members.

The forming sleeves may be inserted into the support element by an automated machine. A preferred automated machine comprises a base and retractable forming sleeves. Using this machine, the open end of the cone is placed partially over the base of the automated machine with the forming sleeves retracted and then the forming sleeves are extended. The support element then is moved toward the cone after the sleeves are extended, and the support element is brought in contact with each forming sleeve in succession until the cone is pleated. Alternatively, the support element may be fixed and the cone and the extended forming sleeves moved toward the support element. Each forming sleeve is then brought into contact in succession with the support element until the cone is pleated.

The invention also provides an alternate method of manufacturing a fluid filter in which the central axes of the cone and the support element are aligned with the open end of the cone closest to the support element. A portion of the cone is inserted into a circular area between two concentric annular members by placing a forming sleeve over the cone and inserting the forming sleeve between the two concentric annular members.

The invention also provides a method of manufacturing a fluid filter comprising: (a) providing an unassembled housing having an integrated cap portion and a generally cylindrical wall portion and a separate base portion; (b) dispensing adhesive into the separate base portion; (c) inserting the support element containing a portion of the cone into the separate base portion and into the adhesive; and (d) attaching the integrated cap portion and generally cylindrical wall portion to the separate base portion. In a preferred embodiment the fluid filter is a blood filter, the integrated cap portion and a generally cylindrical wall portion comprises a blood inlet and a gas vent, and the separate base portion comprises a blood outlet. The adhesive is preferably cured by ultraviolet radiation.

The invention also provides another method of manufacturing a fluid filter comprising: (a) providing an unassembled housing having an integrated cap portion and a generally cylindrical wall portion and a separate base portion; (b) dispensing adhesive into the separate base portion; (c) inserting a filter element into the separate base portion and into the adhesive; and (d) attaching the integrated cap portion and generally cylindrical wall portion to the separate base portion so that the adhesive is disposed between the integrated cap portion and generally cylindrical wall portion and the separate base portion, between the integrated cap portion and generally cylindrical wall portion and the filter element, and between the separate base portion and the filter element. In a preferred embodiment the fluid filter is a blood filter. In another preferred embodiment, the fluid filter is a blood filter, the integrated cap portion and a generally cylindrical wall portion comprises a blood inlet and a gas vent, and the separate base portion comprises a blood outlet. The adhesive is preferably cured by ultraviolet radiation. In a preferred embodiment, the filter element is produced as described above and a portion of the cone is disposed in the adhesive. In another preferred embodiment, the filter element is produced as described above, a first additional portion of the cone is formed over the exterior of the outermost annular member by a forming sleeve inserted outside the outermost annular member, and the first additional portion of the cone is attached within the fluid filter by the adhesive. In another embodiment, the filter element is produced as described above, a first additional portion of the cone is formed over the exterior of the outermost annular member by a forming sleeve inserted outside the outermost annular member, and the first additional portion of the cone is attached within the fluid filter by inserting it within the joint between the integrated cap portion and generally cylindrical wall portion and the separate base portion.

A specific embodiment of the method of manufacturing a fluid filter is described below in the context of a blood filter. The manufacturing process for the blood filter comprises the following steps: (1) assembly of the filter cone 60, (2) pleating the filter cone 60 onto the support element 30, (3) ultrasonically staking (attaching) the filter cone 60 to the support element 30, (4) trimming the pleated cone 60, (5) dispensing the ultraviolet radiation (UV) activatable adhesive into the base portion 16, (6) placing the support element 30 on top of the adhesive, (7) lowering the integrated wall portion 14 and cap portion 12 onto the separate base portion 16 until it locks into the snap joint 17, and (8) curing the UV activatable adhesive.

### 1. Assembly of the Filter Cone 60

The filter screen fabric is cut to size and is seam-bonded so the resulting shape is a hollow three-dimensional cone with a rounded tip. The cone can be fabricated from either one or two pieces of filter screen. The axis of the cone pattern is preferably cut on the bias of the screen threads. This bias cut allows the screen to conform to the shape of the support element without forming wrinkles, creases, or bulges.

A single piece cone may be made from a two-dimensional flat pattern which has the shape of a two-dimensional cone with an arc for the base. The flat piece may be wrapped around a solid three-dimensional cone to form the correct shape and the single seam may be bonded. In a preferred method, the filter screen cone 60 is made from two pieces. The flat pattern 58 for a two piece cone is shown in FIG. 9. The second piece is laid on top of the first piece and the side and tip seams are bonded. Note that the seam 59 is on the outside of the cone with either of these methods of fabrication.

The seams of the filter cone 60 may be bonded by one of several processes. Adhesive strips or sheets may be placed between the two pieces, below the bottom piece, above the top pieces, or a combination of the above. The adhesive is activated (melted) by an energy source such as UV, ultrasonic vibrations, or an impulse heater, and the adhesive is caused to flow such that it contacts both the upper and lower pieces of the cone. The energy source is turned off and the adhesive is allowed to solidify and bond the two pieces of filter screen together. The two pieces could also be bonded with a contact adhesive or fused together without an adhesive by using an energy source to melt the two pieces of filter screen together. For a polyester screen, the preferred energy source is ultrasonic vibrations.

### 2. Pleating the Filter Cone 60 Onto the Support Element 30

As noted above, the filter cone 60 may be pleated manually using a series of concentric cylindrical forming sleeves 80. In a preferred embodiment, the filter cone 60 is pleated by an automated machine.

The filter cone 60 may be manually formed into concentric pleats by shaping the filter cone 60 over the cylindrical support element 30 using cylindrical forming sleeves 80. The support element 30 has a series of concentric annular members 70, 72, 74, and 76, each larger in diameter than the previous one (see FIGS. 6, 7, and 10). Forming sleeves 66, 67, and 68 are sized to fit between the annular members 32 of the support element 30. The first forming sleeve 64 fits inside the first annular member 70 of the support element 30 and the fifth sleeve 69 fits outside the fourth annular member 76 of the element 30. The filter cone 60 may be pleated inside pleat first or outside pleat first.

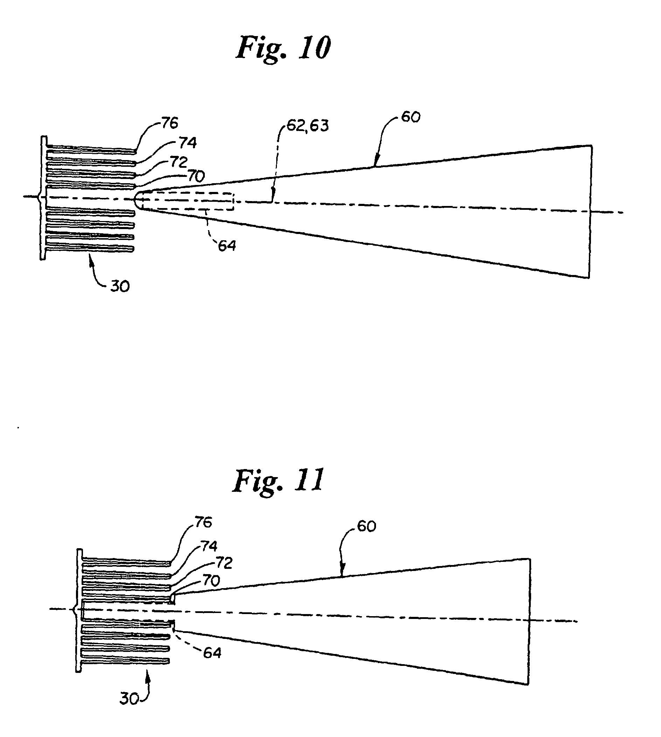

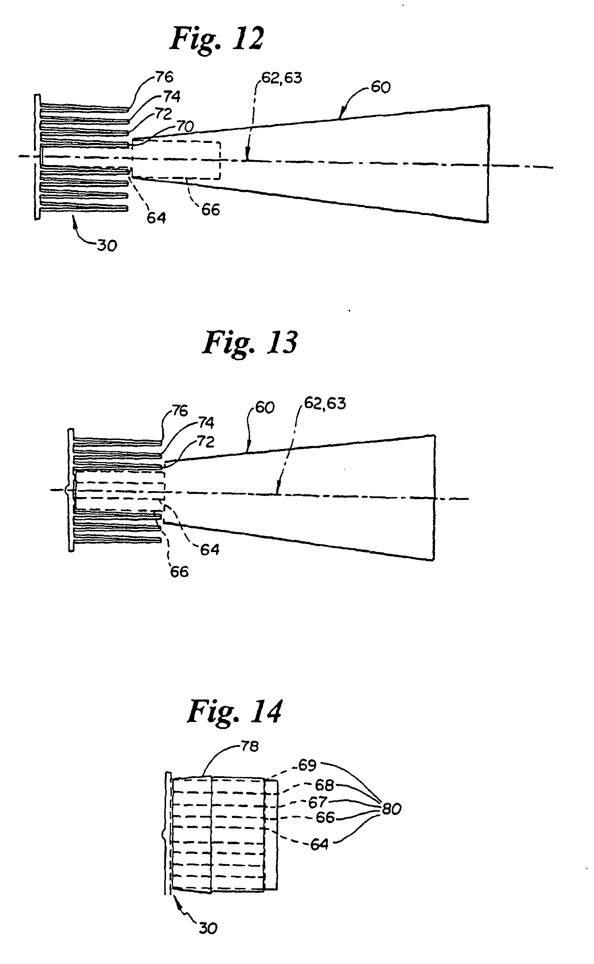

To pleat the filter cone 60 inside pleat first, the following procedure may be used. With the central axis 62 of the cone 60 aligned along the central axis 63 of the support element 30, the tip of the cone 60 is placed toward the support element 30 with the open end of the cone 60 away from the element 30. The first (smallest) forming sleeve 64 is inserted inside the cone 60 (FIG. 10) and used to drive the tip of the cone 60 down into the first (inner) annular member 70 and into the grooves 45 on the surface of the spokes 46 of the support element 30 (FIG. 11). With the first sleeve 64 remaining in place, the next largest sleeve is inserted into the cone 60 (FIG. 12) and used to drive the cone 60 into the space between the annular members 70 and 72 and into the grooves 45 on the spokes 46 (FIG. 13). Note that this process inverts the cone from its orientation of fabrication and places the seam on the inside surface of the pleats, which is desirable because the seam is on the outlet side of the filter screen 60. The seam holds the screen away from the annular members much as the vertical ribs on the annular members perform this function. The cosmetic appearance of the pleats also is improved by having the seam on the outlet side of the filter screen. This process may be performed at either a vertical, horizontal, or sloped orientation as long as the central axes of the filter cone 60 and support element 30 are aligned. The process is continued until the fifth (largest) sleeve 69 is used to pleat the cone 60 over the outside of the support element 30 and into circular groove 47 (FIG. 14). Some extra cone material 78 will extend away from the support element 30 on the outside of the fifth forming sleeve 69.

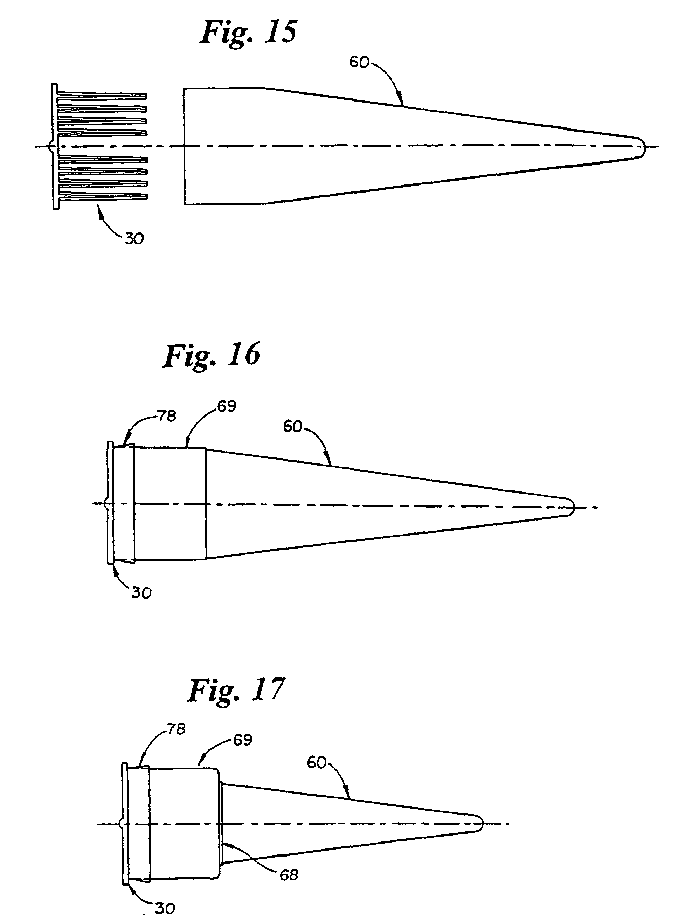

To pleat the filter cone 60 outside pleat first, the following procedure may be used. With the central axis 62 of the cone 60 aligned along the central axis 63 of the support element 30, the open end of the cone 60 is placed toward the support element 30 with the tip of the cone 60 away from the element 30 (FIG. 15). The fifth (largest) sleeve 69 is slipped over the outside of the cone 60 and is used to drive the cone 60 onto the outside of annular member 76 and into circular groove 47 (FIG. 16). With the fifth sleeve 69 remaining in place, the fourth (next largest) sleeve 68 is slipped over the cone 30 and used to drive the cone 30 into the space between annular members 76 and 74 and into the grooves 45 on the surface of the spokes 46 of the support element 30 (FIG. 17). Note that this method does not invert the cone. This process may be performed at either a vertical, horizontal, or sloped orientation as long as the central axes of the filter cone 60 and support element 30 are aligned. The process is continued until the first (smallest) sleeve 64 is used to form the inner pleat.

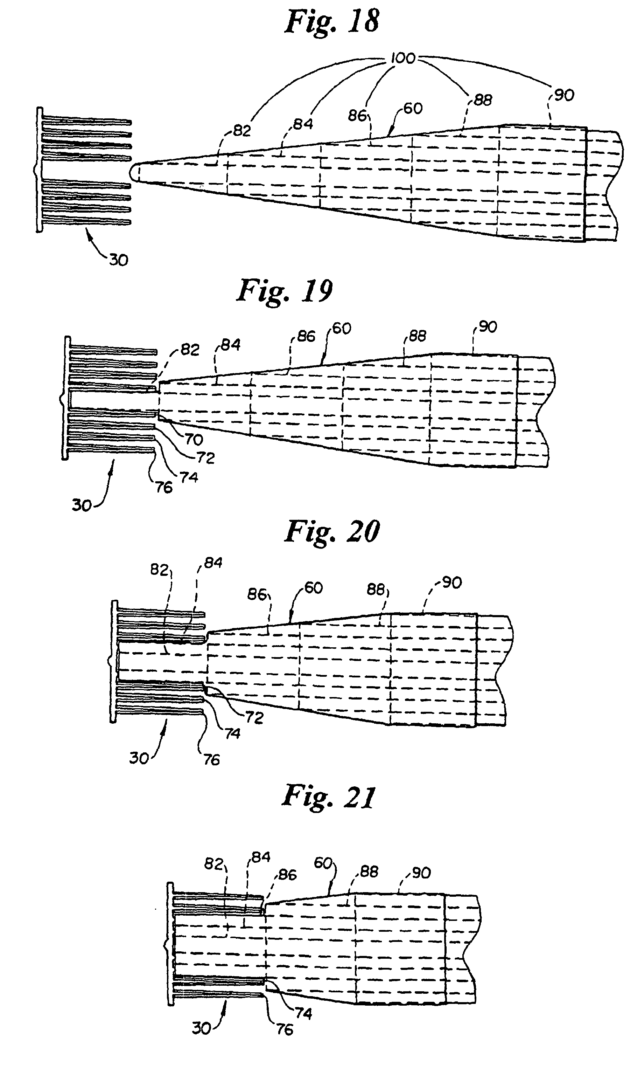

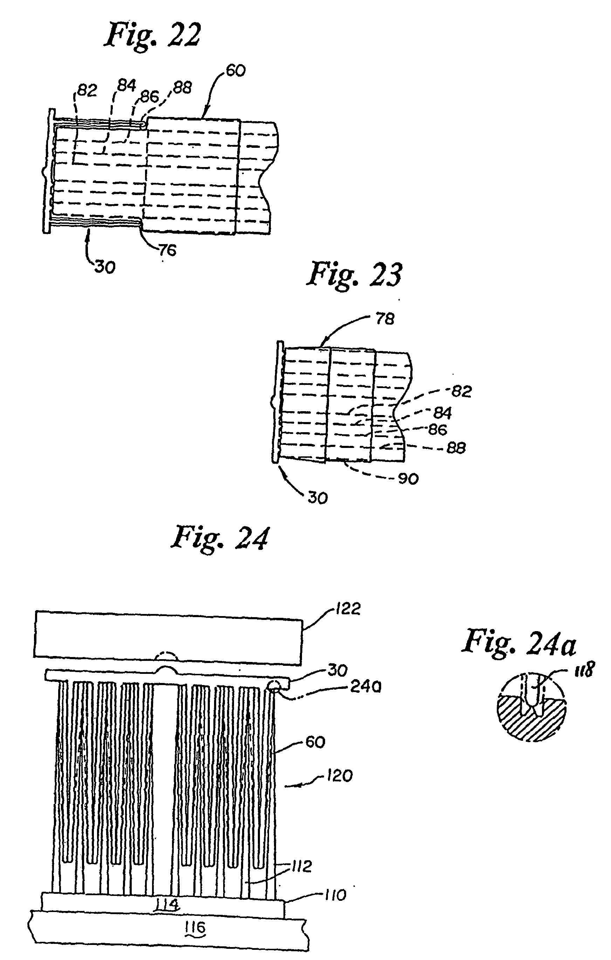

In a preferred embodiment, the filter cone 60 is pleated by an automated pleating machine. The automated pleating machine used to pleat the filter cone 60 uses the inside pleat first method, but a machine could be built to pleat using the outside pleat first method. The pleating machine uses four moving forming sleeves 82, 84, 86, and 88 and one fixed forming sleeve 90. The support element is mounted to a moving fixture which pushes it into the sleeves 100. All the sleeves 100 and the support element 30 are aligned along the same central axis. The first step is to install the support element 30 in its fixture. The second step is to pull the cone 30 over the fixed sleeve 90 with the inner four sleeves 82, 84, 86, and 88 retracted inside the fifth sleeve 90. The inner four sleeves 82, 84, 86, and 88 are then extended into the cone 60 to fill it out (FIG. 18). Next the pleating cycle is initiated and the support element 30 is moved toward the sleeves 100 so as to drive the cone tip down into the first annular member 70 and into the grooves 45 on the surface of the spokes 46 of the support element 30 (FIG. 19). When the first sleeve 82 bottoms out against the spokes 46 on the support element 30, it is pushed backward by the element so that the second sleeve begins to drive the cone 60 into the gap between the first annular member 70 and second annular member 72 of the support element 30 and into the grooves on the spokes 46. Thus, the second pleat is formed (FIG. 20). The pleating process continues (FIGS. 21 and 22) until all four of the moveable sleeves 82, 84, 86, and 88 have been pushed back inside the fifth sleeve 90, the fifth sleeve 90 has been used to pleat the cone 60 over the outside of the support element 30 and into circular groove 47, and all the pleats have been formed (FIG. 23). The support element 30 then is withdrawn from the sleeves 100 which remain retracted. In a preferred embodiment, the support element 30 is rotated as it is withdrawn from the sleeves 100 so there is less chance of the pleats of the pleated cone 60 becoming snagged on one of the sleeves 100.

### 3. Ultrasonically Staking the Filter Cone 60 to the Support Element 30

The support element 30 with a cone 60 pleated onto it is placed upside down in a welding nest 110. The welding nest 110 comprises concentric sleeves 112 attached to a base disk 114 which in turn rests on the table 116 of the ultrasonic welding machine 120 (FIG. 24). The concentric sleeves 112 of the welding nest 110 press the cone 60 against the energy directors 118 on the spokes 46 of the support element 30 in the areas between the annular members 70, 72, 74, and 76, and on the base ring 44 outside annular member 76. A detail of an energy director 118 is shown in the circled portion of FIG. 24. When the ultrasonic welding machine 120 is activated, the horn 122 of the welder 120 is lowered until it presses down on the bottom surface of the base of the support element 30 which is not facing upward (FIG. 24). The horn 122 is excited so it vibrates at a specific frequency and amplitude. The horn 122 in turn vibrates the support element 30 which causes the energy directors 118 to be vibrated against the creases in the pleats of the cone 60. A portion of the support element 30 between the moving energy directors 118 and the cone 60 melts and the cone 60 is embedded in the molten plastic. The vibration is then terminated and the horn 122 is held against the base of the element 30 for a time to allow the molten plastic to harden. The bond of the cone 60 to the element 30 is mechanical. The bond of the cone 30 to the base ring 44 is the most important bond because it forms a seal which prevents blood from passing from the blood inlet chamber 34 to the blood outlet chamber 36 of the filter 10 without going through the filter cone 60.

In a preferred embodiment, the melting point of the support element 30 is lower than that of the cone 60 so that only the portions of the support element 30 near the energy directors 118 melt. The filter screen of the cone 60 remains intact and is not significantly damaged or weakened by the heat or vibration. In a preferred embodiment, the support element 30 is made of ABS plastic and the cone 60 is made of polyester. An energy director 118 facilitates the staking, but the cone also could be staked to the bottom or sides of the base ring 44 of the element 30.

The support element 30 may be made of any appropriate material. Any type of medical grade plastic is preferred. The filter screen of the cone 60 also may be made of any appropriate material. Any type of medical grade plastic filter material is preferred. In one preferred embodiment, the filter screen of the cone 60 is woven from polyester monofilament fiber having a nominal diameter of 30.25 microns. The pores in the filter screen have a nominal size of 38 microns and the nominal percentage of open area is 31 percent.

The cone 60 may be bonded to the support element 30 with an adhesive instead of ultrasonic staking, or in addition to ultrasonic staking. Also, the cone 60 could be staked or bonded to the support element 30 in places other than, or in addition to, the spokes 46 and base ring 44 of the base of the support element 30.

### 4. Trimming the Pleated Cone 60

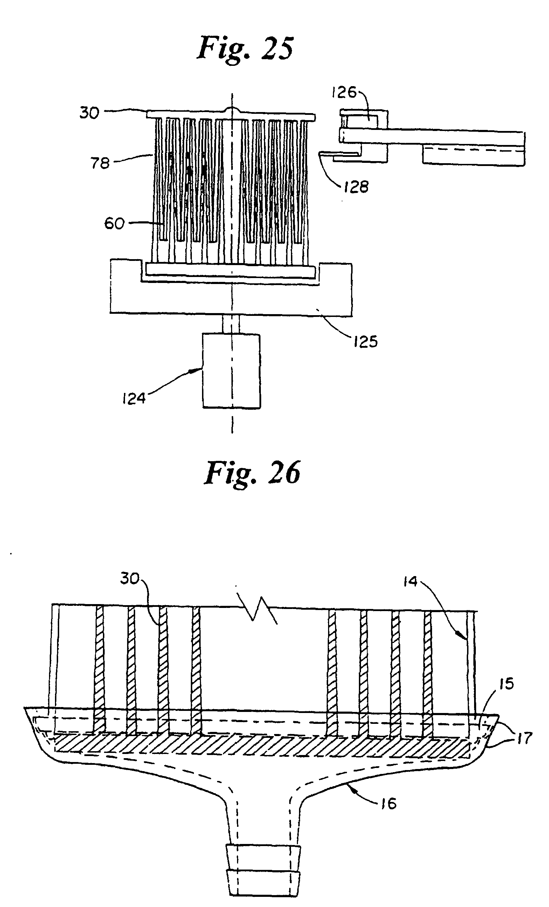

With the support element 30 still located in the welding nest 110, the welding nest 110 is placed in a fixture 125 to trim off the extra cone material 78 which extends beyond the staking point on the support element 30 and wraps around the outside sleeve of the welding nest 110. The fixture 125 rotates the welding nest 110 and thus the pleated cone 60 while a cutting blade 126 and a depth wheel 128 are pressed against the outside surface of the welding nest 110 to trim off the extra cone material 78 (FIG. 25). The fixture 125 is rotated by a motor 124.

### 5. Dispensing the UV Activatable Adhesive Into the Base Portion 16

A separate base portion 16 is positioned on a support fixture. A dispensing needle then moves into a precise position in close proximity to the separate base portion 16 and the adhesive is dispensed through the tip of the needle as the separate base portion 16 rotates below it.

### 6. Placing the Support Element 30 on Top of the Adhesive

An integrated wall portion 14 and cap portion 12 is inserted into a fixture above the separate base portion 16 and the integrated wall portion 14 and cap portion 12 is held in place. A locating arm then moves a guide into position to align the placement of the support element 30 above the separate base portion 16. The element 30 is slid down the guide until it rests in the adhesive.

### 7. Attaching the Wall Portion 14 to the Base Portion 30

The integrated wall portion 14 and cap portion 12 is lowered at a controlled rate onto the separate base portion 16 until the outer lip 15 of the integrated wall portion 14 and cap portion 12 is embedded in the adhesive and captured by the mating components of the snap joint 17 on the separate base portion 16. The support element 30 is pressed into the adhesive and held against the separate base portion 16 by the bottom of the integrated wall portion 14 and cap portion 12. The adhesive seals the gap between the separate base portion 16 and support element 30, and integrated wall portion 14 and cap portion 12 and support element 30. The adhesive also bonds and seals the separate base portion 16 to the integrated wall portion 14 and cap portion 12. Thus, a triple joint is formed (FIG. 26). The assembled filter 10 can now be moved to the next station.

### 8. Curing the UV Activatable Adhesive

The assembled filter 10 is placed on a support fixture. The support fixture lowers the filter 10 into a chamber and a cover door slides over the top of the chamber. A UV emitting bulb is turned on (or exposed by shutters) for a fixed interval to cure the adhesive in the filter 10. The cover door retracts when the light is turned off (or shuttered) and the filter 10 is raised out of the chamber and removed by the operator. The manufacturing process is now complete.

From the foregoing detailed description of specific embodiments of the invention, it is apparent that an improved method of manufacturing a fluid filter has been disclosed. Although particular embodiments of the invention have been disclosed herein in detail, this has been done for the purpose of illustration only, and is not intended to be limiting with respect to the scope of the appended claims, which follow.

## Claims

1. A method of manufacturing a fluid filter element comprising:
a) providing a cone having a tip end and an open end and made of a filter material;
b) providing a support element having a plurality of concentric annular members mounted on a base and having outermost and innermost concentric annular members;
c) aligning the central axes of the cone and the support element with the tip end of the cone closest to the support element;
d) inserting each of a plurality of portions of the cone into separate circular areas between two concentric annular members by placing a plurality of forming sleeves inside the cone and inserting the forming sleeve between the two concentric annular members, the concentric annular members being generally cylindrical and having an interior and an exterior;
e) inserting a portion of the cone into the circular area in the interior of the innermost annular member by a forming sleeve; and
f) forming a portion of the cone over the exterior of the outermost annular member by inserting a forming sleeve outside the outermost annular member.

2. The method of claim 1, wherein the forming sleeve is an annular forming sleeve.

3. The method of claim 1, wherein a portion of the cone is attached to a portion of the base of the support element which is outside the outermost concentric annular member.

4. The method of claim 1, wherein the portion of the cone that is attached to the portion of the base of the support element is attached by ultrasonic vibrations.

5. The method of claim 1, wherein the portion of the cone that is attached to the portion of the base of the support element is attached by inserting a portion of the cone into a groove on the base of the support element.

6. The method of claim 4, wherein the ultrasonic vibrations are conducted through energy directors.

7. The method of claim 1, wherein the support element has a lower melting temperature than the cone.

8. The method of claim 1, wherein the support element has from two to four concentric annular members.

9. The method of claim 1, wherein the fluid filter is a blood filter.

10. The method of claim 1, wherein the forming sleeve is inserted between the two concentric annular members by an automated machine.

11. The method of claim 1, wherein the plurality of forming sleeves are inserted into the separate circular areas by an automated machine.

12. The method of claim 1, wherein each of the forming sleeves is inserted by an automated machine.

13. The method of claim 1, wherein each of the forming sleeves is part of an automated machine comprising a base and the forming sleeves, and wherein the forming sleeves are retractable.

14. The method of claim 13, wherein the open end of the cone is placed partially over the base of the automated machine with the forming sleeves retracted and then the forming sleeves are extended.

15. The method of claim 14, wherein the support element is moved toward the cone after the sleeves are extended and the support element is brought in contact with each forming sleeve in succession until the cone is pleated.

16. The method of claim 15, wherein each portion of the cone that is inserted into the support element is attached to the base of the support element.

17. A method of manufacturing a fluid filter element comprising:
a) providing a cone having a tip end and an open end and made of a filter material;
b) providing a support element having a plurality of concentric annular members mounted on a base and having outermost and innermost concentric annular members;
c) aligning the central axes of the cone and the support element with the open end of the cone closest to the support element;
d) inserting each of a plurality of portions of the cone into separate circular areas between two concentric annular members by placing a plurality of forming sleeves over the cone and inserting the forming sleeve between the two concentric annular members, the concentric annular members being generally cylindrical and having an interior and an exterior;
e) inserting a portion of the cone into the circular area in the interior of the innermost annular member by a forming sleeve; and
f) forming a portion of the cone over the exterior of the outermost annular member by inserting a forming sleeve outside the outermost annular member.

18. The method of claim 1, further comprising:
a) providing an unassembled housing having an integrated cap portion and a generally cylindrical wall portion and a separate base portion;
b) dispensing adhesive into the separate base portion;
c) inserting the support element containing a portion of the cone into the separate base portion and into the adhesive; and
d) attaching the integrated cap portion and generally cylindrical wall portion to the separate base portion.

19. The method of claim 1, further comprising:
a) providing an unassembled housing having an integrated cap portion and a generally cylindrical wall portion and a separate base portion;
b) dispensing adhesive into the separate base portion;
c) inserting the filter element into the separate base portion and into the adhesive; and
d) attaching the integrated cap portion and generally cylindrical wall portion to the separate base portion so that the adhesive is disposed between the integrated cap portion and generally cylindrical wall portion and the separate base portion, between the integrated cap portion and generally cylindrical wall portion and the filter element, and between the separate base portion and the filter element.

20. The method of claim 19, wherein the filter is a blood filter.

## Patentansprüche

1. Verfahren zum Herstellen eines Fluidfilterelements, mit folgenden Schritten:
a) Bereitstellen eines Konus mit einem spitzen Ende und einem offenen Ende, der aus einem Filtermaterial gefertigt ist;
b) Bereitstellen eines Halteelements mit mehreren konzentrischen ringförmigen Elementen auf einer Basis und mit äußersten und Innersten konzentrischen ringförmigen Elementen;
c) Ausrichten der zentralen Achsen des Konus und des Halteelements, wobei das spitze Ende dem Halteelement am nächsten liegt;
d) Einsetzen jedes der mehreren Teile des Konus in separate kreisförmige Bereiche zwischen zwei konzentrischen ringförmigen Elementen durch Platzieren mehrerer Formhülsen in den Konus und Einsetzen der Formhülsen zwischen zwei konzentrische ringförmige Elemente, wobei die konzentrischen ringförmigen Elemente im wesentlichen zylindrisch ausgebildet sind und eine Innenseite und eine Außenseite aufweisen;
e) Einsetzen eines Teils des Konus in den kreisförmigen Bereich im Inneren des innersten ringförmigen Elements mittels einer Formhülse; und
f) Formen eines Teils des Konus über der Außenseite des äußersten ringförmigen Elements durch Einsetzen einer Formhülse außerhalb des äußersten ringförmigen Elements.

2. Verfahren nach Anspruch 1, bei dem die Formhülse eine ringförmige Formhülse ist.

3. Verfahren nach Anspruch 1, bei dem ein Teil des Konus mit einem Teil der Basis des Halteelements, das außerhalb des äußersten konzentrischen ringförmigen Elements angeordnet ist, verbunden ist.

4. Verfahren nach Anspruch 1, bei dem derjenige Teil des Konus, der mit dem Teil der Basis des Halteelements verbunden ist, durch Ultraschallschwingungen verbunden ist.

5. Verfahren nach Anspruch 1, bei dem derjenige Teil des Konus, der mit dem Teil, der Basis des Halteelements verbunden ist, durch Einsetzen eines Teils des Konus in eine Nut in der Basis des Halteelements verbunden ist.

6. Verfahren nach Anspruch 4, bei dem die Ultraschallschwingungen durch Energieleiter geleitet werden.

7. Verfahren nach Anspruch 1, bei dem das Halteelement einen niedrigeren Schmelzpunkt als der Konus hat.

8. Verfahren nach Anspruch 1, bei dem das Halbeelement zwei bis vier konzentrische ringförmige Elemente aufweist.

9. Verfahren nach Anspruch 1, bei dem das Fluidfilter ein Blutfilter ist.

10. Verfahren nach Anspruch 1, bei dem die Formhülse von einer automatisierten Maschine zwischen zwei konzentrische ringförmige Elemente eingesetzt wird.

11. Verfahren nach Anspruch 1, bei dem die mehreren Formhülsen von einer automatisierten Maschine in die separaten ringförmigen Bereiche eingesetzt werden.

12. Verfahren nach Anspruch 1, bei dem jede Formhülse von einer automatisierten Maschine eingesetzt wird.

13. Verfahren nach Anspruch 1, bei dem jede Formhülse Teil einer automatisierten Maschine ist, die eine Basis und die Formhülsen aufweist, wobei dem die Formhülsen zurückfahrbar sind.

14. Verfahren nach Anspruch 13, bei dem das offene Ende des Konus teilweise über der Basis der automatisierten Maschine platziert ist, wobei die Formhülsen zurückgefahren sind und dann ausgefahren werden.

15. Verfahren nach Anspruch 14, bei dem das Halteelement in Richtung des Konus bewegt wird, wenn die Hülsen ausgefahren sind, und das Halteelement nacheinander mit jeder Formhülse in Kontakt gebracht wird ist, bis der Konus gefältelt ist.

16. Verfahren nach Anspruch 15, bei dem jeder Teil des Konus, der in das Halteelement eingesetzt ist, mit der Basis des Halteelements verbunden ist.

17. Verfahren zum Herstellen eines Fluidfilterelements, mit folgenden Schritten:
a) Bereitstellen eines Konus mit einem spitzen Ende und einem offenen Ende, der aus einem Filtermaterial gefertigt ist;
b) Bereitstellen eines Halteelements mit mehreren konzentrischen ringförmigen Elementen auf einer Basis und mit äußersten und innersten konzentrischen ringförmigen Elementen;
c) Ausrichten der zentralen Achsen des Konus und des Halteelements, wobei das offene Ende dem Halteelement am nächsten liegt;
d) Einsetzen jedes der mehreren Teile des Konus in separate kreisförmige Bereiche zwischen zwei konzentrischen ringförmigen Elementen durch Platzieren mehrerer Formhülsen über den Konus und Einsetzen der Formhülsen zwischen zwei konzentrische ringförmige Elemente, wobei die konzentrischen ringförmigen Elemente im wesentlichen zylindrisch ausgebildet sind und eine Innenseite und eine Außenseite aufweisen;
e) Einsetzen eines Teils des Konus in den kreisförmigen Bereich im Inneren des innersten ringförmigen Elements mittels einer Formhülse; und
f) Formen eines Teils des Konus über der Außenseite des äußersten ringförmigen Elements durch Einsetzen einer Formhülse außerhalb des äußersten ringförmigen Elements.

18. Verfahren nach Anspruch 1, ferner mit folgenden Schritten:
a) Bereitstellen eines nicht zusammengesetzten Gehäuses mit einem einstückig angeformten Kappenteil und einem im wesentlichen zylindrischen Wandteil und einem separaten Basisteil;
b) Platzieren von Kleber in den separaten Basisteil;
c) Einsetzen des Halteelements mit einem Teil des Konus in den separaten Basisteil und in den Kleber; und
d) Verbinden des einstückig angeformten Kappenteils und des im wesentlichen zylindrischen Wandteils mit dem separaten Basisteil.

19. Verfahren nach Anspruch 1, ferner mit folgenden Schritten:
a) Bereitstellen eines nicht zusammengesetzten Gehäuses mit einem einstückig angeformten Kappenteil und einem im wesentlichen zylindrischen Wandteil und einem separaten Basisteil;
b) Platzieren von Kleber in den separaten Basisteil;
c) Einsetzen des Filterelements in den separaten Basisteil und in den Kleber; und
d) Verbinden des einstückig angeformten Kappenteils und des im wesentlichen zylindrischen Wandteils mit dem separaten Basisteil, so dass sich der Kleber zwischen dem einstückig angeformten Kappenteil und dem im wesentlichen zylindrischen Wandteil und dem separaten Basisteil, zwischen dem einstückig angeformten Kappentell und dem im wesentlichen zylindrischen Wandteil und dem Filterelement, und zwischen dem separaten Basisteil und dem Filterelement befindet.

20. Verfahren nach Anspruch 19, bei dem das Filter ein Blutfilter ist.

## Revendications

1. Procédé de fabrication d'un élément de filtre de fluide, comprenant :
a) la disposition d'un cône ayant une extrémité de bout et une extrémité ouverte et formé d'un matériau de filtre,
b) la disposition d'un élément de support ayant plusieurs organes concentriques annulaires montés sur une base et ayant des organes annulaires concentriques le plus externe et le plus interne,
c) l'alignement des axes centraux du cône et de l'élément de support sur l'extrémité de bout du cône la plus proche de l'élément de support,
d) l'insertion de chacune des parties de cône dans des régions circulaires séparées formées entre deux organes annulaires concentriques par disposition de plusieurs manchons de mise en forme à l'intérieur du cône et insertion du manchon de mise en forme entre deux organes annulaires concentriques, les organes annulaires concentriques ayant une forme générale cylindrique et comprenant une partie intérieure et une partie extérieure,
e) l'insertion d'une partie du cône dans la région circulaire formée à l'intérieur de l'organe annulaire le plus interne par un manchon de mise en forme, et
f) la mise en forme d'une partie du cône à l'extérieur de l'organe annulaire le plus externe par insertion d'un manchon de mise en forme à l'extérieur de l'organe annulaire le plus externe.

2. Procédé selon la revendication 1, dans lequel le manchon de mise en forme est un manchon annulaire de mise en forme.

3. Procédé selon la revendication 1, dans lequel une partie du cône est fixée à une partie de la base de l'élément de support qui est en dehors de l'organe annulaire concentrique le plus externe.

4. Procédé selon la revendication 1, dans lequel la partie du cône qui est fixée à la partie de la base de l'élément de support est fixée par des vibrations ultrasonores.

5. Procédé selon la revendication 1, dans lequel la partie du cône qui est fixée à la partie de la base de l'élément de support est fixée par insertion d'une partie du cône dans une gorge de la base de l'élément de support.

6. Procédé selon la revendication 4, dans lequel les vibrations ultrasonores sont conduites par des organes directeurs d'énergie.

7. Procédé selon la revendication 1, dans lequel l'élément de support a une température de fusion inférieure à celle du cône.

8. Procédé selon la revendication 1, dans lequel l'élément de support a deux à quatre organes annulaires concentriques.

9. Procédé selon la revendication 1, dans lequel le filtre de fluide est un filtre de sang.

10. Procédé selon la revendication 1, dans lequel le manchon de mise en forme est inséré entre deux organes annulaires concentriques par une machine automatique.

11. Procédé selon la revendication 1, dans lequel les manchons de mise en forme sont insérés dans les régions circulaires séparés par une machine automatique.

12. Procédé selon la revendication 1, dans lequel chacun des manchons de mise en forme est inséré par une machine automatique.

13. Procédé selon la revendication 1, dans lequel chacun des manchons de mise en forme fait partie d'une machine automatique comprenant une base et les manchons de mise en forme, et les manchons de mise en forme sont rétractables.

14. Procédé selon la revendication 13, dans lequel l'extrémité ouverte du cône est disposée partiellement au-dessus de la base de la machine automatique avec les manchons de mise en forme rétractés et les manchons de mise en forme sont alors allongés.

15. Procédé selon la revendication 14, dans lequel l'élément de support est déplacé vers le cône après que les manchons ont été allongés et l'élément de support est placé au contact de chaque manchon de mise en forme successivement jusqu'à ce que le cône soit plissé.

16. Procédé selon la revendication 15, dans lequel chaque partie du cône qui est insérée dans l'élément de support est fixée à la base de l'élément de support.

17. Procédé de fabrication d'un élément de filtre de fluide, comprenant :
a) la disposition d'un cône ayant une extrémité de bout et une extrémité ouverte et formé d'un matériau de filtre,
b) la disposition d'un élément de support ayant plusieurs organes annulaires concentriques montés sur une base et ayant des organes annulaires concentriques le plus externe et le plus interne,
c) l'alignement des axes centraux du cône et de l'élément de support sur l'extrémité ouverte du cône la plus proche de l'élément de support,
d) l'insertion de chacune des parties du cône dans des régions circulaires séparées formées entre deux organes annulaires concentriques par disposition de plusieurs manchons de mise en forme sur le cône et insertion du manchon de mise en forme entre les deux organes annulaires concentriques, les organes annulaires concentriques ayant une forme générale cylindrique et possédant une partie intérieure et une partie extérieure,
e) l'insertion d'une partie du cône dans la région circulaire à l'intérieur de l'organe circulaire le plus interne par un manchon de mise en forme, et
f) la mise en forme d'une partie du cône sur l'extérieur de l'organe annulaire le plus externe par insertion d'un manchon de mise en forme à l'extérieur de l'organe annulaire le plus externe.

18. Procédé selon la revendication 1, comprenant en outre :
a) la disposition d'un boîtier non assemble ayant une partie de capuchon et une partie de paroi de forme générale cylindrique qui sont intégrées et une partie séparée de base,
b) la distribution d'un adhésif dans la partie séparée de base,
c) l'insertion de l'élément de support contenant une partie du cône dans la partie séparée de base et dans l'adhésif, et
d) la fixation de la partie de capuchon et de la partie de paroi de forme générale cylindrique qui sont intégrées sur la partie séparée de base.

19. Procédé selon la revendication 1, comprenant en outre :
a) la disposition d'un boîtier non assemblé ayant une partie de capuchon et une partie de paroi de forme générale cylindrique intégrée et une partie séparée de base,
b) la distribution d'un adhésif dans la partie séparée de base,
c) l'insertion de l'élément de filtre dans la partie séparée de base et dans l'adhésif, et
d) la fixation de la partie de capuchon et de la partie de paroi de forme générale cylindrique qui sont intégrées à la partie séparée de base de manière que l'adhésif soit placé entre la partie de capuchon et la partie de paroi de forme générale cylindrique qui sont intégrées et la partie séparée de base, entre la partie de capuchon et la partie de paroi de forme générale cylindrique qui sont intégrées et l'élément de filtre et entre la partie séparée de base et l'élément de filtre.

20. Procédé selon la revendication 19, dans lequel le filtre est un filtre de sang.
